# EUROPEAN PATENT APPLICATION

(11) **EP 2 377 540 A1**
(43) Date of publication of application: **19.10.2011**
(21) Application number: 10003613.6
(22) Date of filing: 31.03.2010
(51) Int. Cl.: A61K 31/565, A61K 47/14, A61K 9/70

(54) **Transdermal patch containing 17-deacetyl norgestimate**

(71) Applicant: Hexal AG, 83607 Holzkirchen (DE)
(72) Inventor: Helfrich, Michael, 83607 Holzkirchen (DE); Grundsteiner, Sigrid, 83607 Holzkirchen (DE)
(74) Representative: Bohmann, Armin K.

(57) **Abstract**

The present invention is related to an enhancer consisting of a monoester or diester of a hydroxy dicarboxylic acid, for use in the transdermal administration of a pharmaceutically active agent to a subject.

## Description

The present invention is related to an enhancer for use in transdermal administration of a pharmaceutically active agent to a subject, a transdermal delivery system comprising such enhancer, a transdermal delivery system for preventing ovulation in a woman, a transdermal delivery system for providing hormone replacement therapy in a woman, and the use of the enhancer in the manufacture of a medicament and a pharmaceutical formulation.

Transdermal delivery systems and more specifically transdermal therapeutic systems which are also referred to herein as TTS, such as transdermal patches, have been proven to be advantageous in the administration and delivery of pharmaceutically active agents. One of the reasons for this is that transdermal delivery systems avoid hepatic metabolization of the pharmaceutically active agent which is frequently observed upon oral administration of a pharmaceutically active agent. As a consequence, upon administration of a pharmaceutically active agent through a transdermal delivery system the liver is relieved and gastrointestinal side-effects are avoided. Additionally, compared to a non-transdermal administration, usually less of the pharmaceutically active agent is required so as to have the same effect. Furthermore, transdermal delivery systems provide a more constant blood level of the pharmaceutically active agent as said agent is immediately effective in a systemic manner upon permeation through the skin. Finally, transdermal delivery systems increase patients' compliance due to their easy and convenient application.

Transdermal therapeutic systems have been shown to be particular useful for preventing ovulation in a woman and for providing hormone replacement therapy in a woman. In both cases, a combination of norelgestromin and ethinyl estradiol is preferably used.

Norelgestromin which is also referred to as 17-deacetyl norgestimate (13-ethyl-17-hydroxy-18,19-dinor-17-alpha-pregn-4-en-20-yn-3-one oxime) is a member of the gestagens. Norelgestromin inhibits the release of the luteinising hormone and is thus inhibiting ovulation. Compared to its prodrug norgestimate and its other metabolites such as 3-keto-norgestimate and levonorgestrel, norelgestromin advantageously only shows a minor androgenic effect.

Estrogens inhibit the secretion of the follicle-stimulating hormone (FSH) and thus inhibit ovulation. The group of estrogens comprises, among others, 17-13-estradiol and ethinyl estradiol.

A particular form of transdermal therapeutic systems are matrix-controlled transdermal therapeutic systems which are also referred to as matrix TTS. Such matrix-controlled transdermal therapeutic systems typically comprise a backing layer which is impermeable for the pharmaceutically active agent, one or several matrix layers, and a release liner. The matrix layer(s) can be self-adhesive or can be coated with an adhesive.

International patent application WO 96/40355 discloses a transdermal patch for the administration of norelgestromin alone or in combination with an estrogen. Such patch contains a backing layer which is impermeable for the drug and a matrix layer which contains the drug and polyisobutylene and/or silicone as pressure-sensitive adhesive. The patch uses as enhancer esters of lactic acid and C₁₂ - C₁₈ aliphatic alcohols, oleic acid and polyethylene glycol monolaurate. WO 96/40355 recites as enhancers lauryl lactate and isopropyl palmitate.

US 20040053901 discloses transdermal therapeutic systems for the administration of levonorgestrel. The systems use an enhancer composition which comprises a combination of a pharmaceutically acceptable organic solvent, a fatty (C₈ - C₂₀) alcohol ester of a hydroxyl acid, a lower (C₁- C₄) alkyl ester of a hydroxyl acid and a C₆ - C₁₈ saturated or unsaturated fatty acid.

The problem underlying the present invention is to provide an enhancer which provides for a higher *in vivo* flux of pharmaceutically active agents such as a hormone and ethinyl estradiol in particular when used in a transdermal therapeutic system such as a transdermal patch.

A further problem underlying the present invention is to provide a transdermal therapeutic system such as a transdermal patch which shows a high *in vivo* flux of a pharmaceutically active agent such as a hormone and ethinyl estradiol in particular.

Another further problem underlying the present invention is to provide a transdermal therapeutic system such as a transdermal patch containing a content of a pharmaceutically active agent such as a hormone and ethinyl estrogen in particular, which contains a reduced amount of said pharmaceutically active agent while providing a serum level of said pharmaceutically active agent comparable to the one observed with transdermal therapeutic systems of the prior art containing more of said pharmaceutically active agent.

A still further problem underlying the instant invention is to provide a transdermal therapeutic system such as a transdermal patch containing a content of a pharmaceutically active agent such as a hormone and ethinyl estrogen in particular, which contains an amount of said pharmaceutically active agent providing a serum level of said pharmaceutically active agent in a subject to which the transdermal therapeutic system is applied, comparable to the one observed with transdermal therapeutic systems of the prior art containing said pharmaceutically active agent whilst allowing for a more comprehensive overall flux or transfer of said pharmaceutically active agent from the transdermal therapeutic system to the subject so that upon removal of the transdermal therapeutic system from the subject, the transdermal therapeutic system contains less of said pharmaceutically active agent than the transdermal therapeutic system of the prior art.

These and other problems are solved by the subject matter of the attached claims.

Furthermore, these and other problems are solved, in a first aspect, which is also the first embodiment of the first aspect, by an enhancer consisting of a monoester or diester of a hydroxy dicarboxylic acid, for use in the administration, preferably transdermal administration, of a pharmaceutically active agent to a subject.

In a second embodiment of the first aspect which is also an embodiment of the first embodiment of the first aspect, the hydroxy dicarboxylic acid contains one or more hydroxy groups.

In a third embodiment of the first aspect which is also an embodiment of the second embodiment of the first aspect, the hydroxy dicarboxylic acid contains n hydroxyl groups, wherein n is any integer from 1 to 5, preferably 1 to 2 and more preferably 1.

In a fourth embodiment of the first aspect which is also an embodiment of the first, second and third embodiment of the first aspect, the hydroxy dicarboxylic acid is of formula (I)

R₁OOC-(CHOH)ₓ-(CH₂)_{y}-(CH₂OH)_{z}-COOR₂ (I)

wherein x and z are each and independently any integer from 0 to 5, whereby if x = z, x and z are # 0 ,
preferably x is any integer from 1 -5 ,
more preferably x is 1 or 2, and z is 0 ;
y is any integer form 0 to 10, preferably 1-5 , most preferably 1; and
R₁ and R₂ are each and independently selected from the group consisting of H, C₁-C₈, C₈-C₁₈, wherein at least one of R1 and R2 has to be different from H.

In a fifth embodiment of the first aspect which is also an embodiment of the fourth embodiment of the first aspect, x is 1 or 2, z is 0 and y is 1.

In a sixth embodiment of the first aspect which is also an embodiment of the fourth and fifth embodiment of the first aspect, C₁-C₈ and C₈-C₁₈ are each and independently linear or branched.

In a seventh embodiment of the first aspect which is also an embodiment of the fourth, fifth and sixth embodiment of the first aspect, R1 and R2 are each and independently a mixture of a branched C 12 and C 13 alcohol of formula (II) 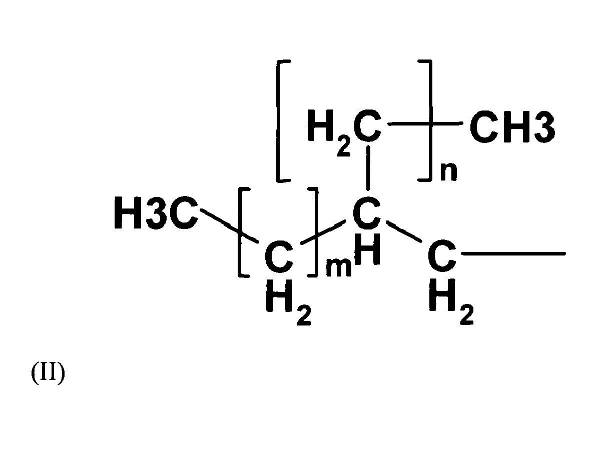 wherein m + n is either 8 or 9.

In an eighth embodiment of the first aspect which is also an embodiment of the fourth, fifth, sixth and seventh embodiment of the first aspect, R1 and R2 are the same.

In a ninth embodiment of the first aspect which is also an embodiment of the fourth, fifth, sixth and seventh embodiment of the first aspect, R1 and R2 are different.

In a tenth embodiment of the first aspect which is also an embodiment of the first, second, third, fourth, fifth, sixth, seventh, and eighth embodiment of the first aspect, the hydroxy dicarboxylic acid is selected from the group comprising 2-hydroxybutanedioic acid and 2,3-dihydroxybutanedioic acid.

In an eleventh embodiment of the first aspect which is also an embodiment of the first, second, third, fourth, fifth, sixth, seventh, eighth, ninth and tenth embodiment of the first aspect, the enhancer is the diester of 2-hydroxybutanedioic acid and a mixture of a branched C12 and C 13 alcohol of formula (II) 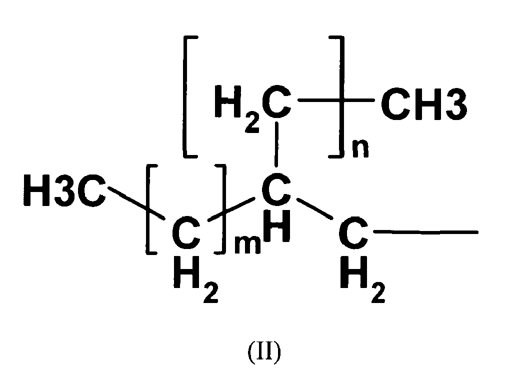 wherein m + n is either 8 or 9.

In a twelfth embodiment of the first aspect which is also an embodiment of the first, second, third, fourth, fifth, sixth, seventh, eighth, ninth, tenth and eleventh embodiment of the first aspect, the enhancer is Cosmacol^{®} EMI.

In a second aspect, which is also the first embodiment of the second aspect, the problems underlying the present invention are solved by an enhancer comprising a monoester or diester of a hydroxy dicarboxylic acid, for use in the administration of a pharmaceutically active agent to a subject.

In a second embodiment of the second aspect which is also an embodiment of the first embodiment of the second aspect, the monoester or diester of a hydroxy dicarboxylic acid is the monoester or diester of a hydroxy dicarboxylic acid as defined in connection with any embodiment of the first aspect and more specifically any one of embodiments one to twelve of the first aspect.

In a third embodiment of the second aspect which is also an embodiment of the first and second embodiment of the second aspect and a 13^{th} embodiment of the first aspect which is also an embodiment of the first, second, third, fourth, fifth, sixth, seventh, eighth, ninth, tenth, eleventh and twelfth embodiment of the first aspect, the administration is transdermal administration.

In a fourth embodiment of the second aspect which is also an embodiment of the first, second and third embodiment of the second aspect and a 14^{th} embodiment of the first aspect which is also an embodiment of the first, second, third, fourth, fifth, sixth, seventh, eighth, ninth, tenth, eleventh, twelfth and 13^{th} embodiment of the first aspect, the enhancer is a skin permeation enhancer.

In a fifth embodiment of the second aspect which is also an embodiment of the first, second, third and fourth embodiment of the second aspect and a 15^{th} embodiment of the first aspect which is also an embodiment of the first, second, third, fourth, fifth, sixth, seventh, eighth, ninth, tenth, eleventh, twelfth, 13^{th} and 14^{th} embodiment of the first aspect, the pharmaceutically active agent comprises at least one hormone.

In a sixth embodiment of the second aspect which is also an embodiment of the fifth embodiment of the second aspect and a 16^{th} embodiment of the first aspect which is also an embodiment of the 15^{th} embodiment of the first aspect, the at least one hormone is one selected from the group comprising an estrogen.

In a seventh embodiment of the second aspect which is also an embodiment of the sixth embodiment of the second aspect and a 17^{th} embodiment of the first aspect which is also an embodiment of the 16^{th} embodiment of the first aspect, the at least one hormone is one selected from the group consisting of 17-deacetyl norgestimate, ethinyl estradiol and 17-ß-estradiol.

In an eighth embodiment of the second aspect which is also an embodiment of the fifth embodiment of the second aspect and an 18^{th} embodiment of the first aspect which is also an embodiment of the 15^{th} embodiment of the first aspect, the pharmaceutically active agent comprises or consists of a combination of 17-deacetyl norgestimate and ethinyl estradiol.

In a ninth embodiment of the second aspect which is also an embodiment of the first, second, third, fourth, fifth, sixth, seventh and eighth embodiment of the second aspect and a 19^{th} embodiment of the first aspect which is also an embodiment of the first, second, third, fourth, fifth, sixth, seventh, eighth, ninth, tenth, eleventh, twelfth, 13^{th}, 14^{th}, 15^{th}, 16^{th}, 17^{th} and 18^{th} embodiment of the first aspect, the enhancer is for use in a method for preventing ovulation in a woman.

In a tenth embodiment of the second aspect which is also an embodiment of the first, second, third, fourth, fifth, sixth, seventh, eighth and ninth embodiment of the second aspect and a 20^{th} embodiment of the first aspect which is also an embodiment of the first, second, third, fourth, fifth, sixth, seventh, eighth, ninth, tenth, eleventh, twelfth, 13^{th}, 14^{th}, 15^{th}, 16^{th}, 17^{th}, 18^{th} and 19^{th} embodiment of the first aspect, the enhancer is for use in a method of providing hormone replacement therapy in a woman.

In a third aspect, which is also the first embodiment of the third aspect, the problems underlying the present invention are solved by a transdermal delivery system comprising
a) a backing layer,
b) a matrix layer underlying the backing layer, the matrix layer comprising a pharmaceutically active agent, a skin permeation enhancer and preferably at least one adhesive,
wherein the skin permeation enhancer is an enhancer as defined in any one of preceding embodiments.

In a second embodiment of the third aspect which is also an embodiment of the first embodiment of the third aspect, the transdermal delivery system further comprises
c) a release liner.

In a third embodiment of the third aspect which is also an embodiment of the first and second embodiment of the third aspect, the transdermal delivery system further comprises at least one adhesive layer, preferably at least one pressure sensitive adhesive layer.

In a fourth embodiment of the third aspect which is also an embodiment of the third embodiment of the third aspect, the adhesive is part of the matrix layer.

In a fifth embodiment of the third aspect which is also an embodiment of the first, second, third and fourth embodiment of the third aspect, the transdermal delivery system is a transdermal patch.

In a sixth embodiment of the third aspect which is also an embodiment of the first, second, third, fourth and fifth embodiment of the third aspect, the content of the skin permeation enhancer is about 1 to 20 wt %, preferably about 5 to 10 wt% and more preferably about 7.5 wt % of the matrix layer.

In a seventh embodiment of the third aspect which is also an embodiment of the third, fourth, fifth and sixth embodiment of the third aspect, the adhesive is about 20 to 90 wt %, preferably about 30 to 80 wt % and more preferably about 50 to 70 wt % of the matrix layer.

In an eighth embodiment of the third aspect which is also an embodiment of the first, second, third, fourth, fifth, sixth and seventh embodiment of the third aspect, the pharmaceutically active agent is a hormone, preferably an estrogen and more preferably ethinyl estradiol or 17-ß-estradiol.

In a ninth embodiment of the third aspect which is also an embodiment of the first, second, third, fourth, fifth, sixth and seventh embodiment of the third aspect, the system is for preventing ovulation in a woman.

In a tenth embodiment of the third aspect which is also an embodiment of the ninth embodiment of the third aspect, the pharmaceutically active agent is a combination of 17-deacetyl norgestimate and ethinyl estradiol.

In an eleventh embodiment of the third aspect which is also an embodiment of the ninth and tenth embodiment of the third aspect, the amount of 17-deacetyl norgestimate administered through the system is about 100 - 300 µg/day, preferable about 150 µg/day.

In a twelfth embodiment of the third aspect which is also an embodiment of the ninth, tenth and eleventh embodiment of the third aspect, the amount of ethinyl estradiol administered through the system is about 10 - 35 µg/day, preferable about 20 µg/day.

In a 13^{th} embodiment of the third aspect which is also an embodiment of the ninth, tenth, eleventh and twelfth embodiment of the third aspect, the transdermal delivery system is a patch, preferably a transdermal patch, whereby the patch is intended to be worn for about 1 to 7 days.

In a 14^{th} embodiment of the third aspect which is also an embodiment of the ninth, tenth, eleventh, twelfth and 13^{th} embodiment of the third aspect, the system is a patch of about 10 to 50 cm².

In a 15^{th} embodiment of the third aspect which is also an embodiment of the ninth, tenth, eleventh, twelfth, 13^{th} and 14^{th} embodiment of the third aspect, the system is for use starting from day 1 of the menstruation cycle to day 21 of the menstruation cycle.

In a 16^{th} embodiment of the third aspect which is also an embodiment of the first, second, third, fourth, fifth, sixth and seventh embodiment of the third aspect, the system is for providing hormone replacement therapy in a woman.

In a 17^{th} embodiment of the third aspect which is also an embodiment of the 16^{th} embodiment of the third aspect, the pharmaceutically active agent is a combination of 17-deacetyl norgestimate and ethinyl estradiol.

In an 18^{th} embodiment of the third aspect which is also an embodiment of the 16^{th} and 17^{th} embodiment of the third aspect, the amount of 17-deacetyl norgestimate administered through the system is about 150 - 350 µg/day, preferable about 175 - 300 µg/day.

In a 19^{th} embodiment of the third aspect which is also an embodiment of the 16^{th}, 17^{th} and 18^{th} embodiment of the third aspect, preferably of the 18^{th} embodiment of the third aspect, the amount of ethinyl estradiol administered through the system is about 5 - 45 µg/day, preferable about 10 to 35 µg/day.

In a 20^{th} embodiment of the third aspect which is also an embodiment of the 16^{th} embodiment of the third aspect, the pharmaceutically active agent is a combination of 17-deacetyl norgestimate and 17-ß-estradiol.

In a 21^{st} embodiment of the third aspect which is also an embodiment of the 20^{th} embodiment of the third aspect, the amount of 17-deacetyl norgestimate administered through the system is about 150 - 350 µg/day, preferable about 175 - 300 µg/day.

In a 22^{nd} embodiment of the third aspect which is also an embodiment of the 20^{th} and 21^{st} embodiment of the third aspect, preferably of the 21^{st} embodiment of the third aspect, amount of 17-ß-estradiol administered through the system is about 20 - 175 µg/day, preferable about 30 to 150 µg/day.

In a 23^{rd} embodiment of the third aspect which is also an embodiment of the 16^{th}, 17^{th}, 18^{th}, 19^{th}, 20^{th}, 21^{st} and 22^{nd} embodiment of the third aspect, the transdermal delivery system is a patch, whereby the patch is intended to be worn for about 1 to 7 days.

In a 24^{th} embodiment of the third aspect which is also an embodiment of the 16^{th}, 17^{th}, 18^{th}, 19^{th}, 20^{th}, 21^{st}, 22^{nd} and 23^{rd} embodiment of the third aspect, the system is a patch of about 10 to 50 cm².

In a 25^{th} embodiment of the third aspect which is also an embodiment of the 16^{th}, 17^{th}, 18^{th}, 19^{th}, 20^{th}, 21^{st}, 22^{nd}, 23^{rd} and 24^{th} embodiment of the third aspect, the system is for use for the duration of the hormone replacement therapy.

In a 26^{th} embodiment of the third aspect which is also an embodiment of the first, second, third, fourth, fifth, sixth, seventh, eighth, ninth, tenth, eleventh, twelfth, 13^{th}, 14^{th}, 15^{th}, 16^{th}, 17^{th}, 18^{th}, 19^{th}, 20^{th}, 21^{st}, 22^{nd}, 23^{rd}, 24^{th} and 25^{th} embodiment of the third aspect, the transdermal delivery system comprises:
a) a backing layer,
b) a matrix layer underlying the backing layer; and
c) a release liner,
wherein the matrix layer contains ethinyl estradiol, 17-deacetyl norgestimate, Cosmacol EMI, an adhesive, and optionally cross-linked polyvinylpyrrolidon, wherein preferably the adhesive is polyisobutylene.

In a 27^{th} embodiment of the third aspect which is also an embodiment of the first, second, third, fourth, fifth, sixth, seventh, eighth, ninth, tenth, eleventh, twelfth, 13^{th}, 14^{th}, 15^{th}, 16^{th}, 17^{th}, 18^{th}, 19^{th}, 20^{th}, 21^{st}, 22^{nd}, 23^{rd}, 24^{th} and 25^{th} embodiment of the third aspect, the transdermal delivery system comprises:
a) a backing layer,
b) a matrix layer underlying the backing layer; and
c) a release liner,
wherein the matrix layer comprises ethinyl estradiol, 17-deacetyl norgestimate, Cosmacol EMI, Kollidon CLM and Durotak 87-608A, wherein the amount of ethinyl estradial contained in the matrix layer is a pharmaceutically active amount of ethinyl estradiol of about 0.75 mg/20 cm² or less, preferably of about 0.65 mg/20 cm² and more preferably of about 0.6 mg/20 cm², and wherein the surface weight of the transdermal delivery system is 75 g/m².

In a 28^{th} embodiment of the third aspect which is also an embodiment of the first, second, third, fourth, fifth, sixth, seventh, eighth, ninth, tenth, eleventh, twelfth, 13^{th}, 14^{th}, 15^{th}, 16^{th}, 17^{th}, 18^{th}, 19^{th}, 20^{th}, 21^{st}, 22^{nd}, 23^{rd}, 24^{th}, 25^{th}, 26^{th} and 27^{th} embodiment of the third aspect, the transdermal delivery system comprises:
a) a backing layer,
b) a matrix layer underlying the backing layer; and
c) a release liner,
wherein the matrix layer contains

| Compound | Amount [mg/20 cm of the transdermal delivery system] |
|---|---|
| Ethinyl estradiol | 0.75 |
| 17-deacetyl norgestimate | 6 |
| COSMACOL ^{®} EMI | 11.25 |
| Kollidon CLM | 30 |
| Polyisobutylene, preferably Durotak 87-608A | 102^{*} |

| | |
|---|---|
| *expressed as solid matter content of the solution; solvents are removed during manufacturing | |

and wherein the surface weight of the transdermal delivery system is 75 g/m².

In a fourth aspect, which is also the first embodiment of the fourth aspect, the problems underlying the present invention are solved by the use of an enhancer according to any embodiment of the first and second aspect, more preferably any one of embodiments 1 to 20 of the first aspect and any one of embodiments 1 to 10 of the second aspect, in or for the manufacture of a medicament or a pharmaceutical formulation.

In a second embodiment of the fourth aspect the medicament or pharmaceutical formulation is a transdermal delivery patch comprising at least one pharmaceutically active agent.

In a further embodiment of the first and the second aspect which is also an embodiment of each and any embodiment of the first and the second aspect, the enhancer is different from esters of lactic acid and C₁₂ - C₁₈ aliphatic alcohols, oleic acid and propylene glycol monolaurate or lauryl lactate.

In a further embodiment of the first and the second aspect which is also an embodiment of each and any embodiment of the first and the second aspect, the enhancer is different from diesters of a dicarboxylic acid such as dibutyl sebacate, esters of fatty acids such as isopropyl palmitate and esters of polyhydric alcohols such as propylene glycol stearate.

In a still further embodiment of the first and the second aspect which is also an embodiment of each and any embodiment of the first and the second aspect, the enhancer is different from an enhancer composition comprising a combination of a pharmaceutically acceptable organic solvent, a fatty (C₈ - C₂₀) alcohol ester of a hydroxyl acid, a lower (C₁- C₄) alkyl ester of a hydroxyl acid and a C₆ - C₁₈ saturated or unsaturated fatty acid.

The present inventors have surprisingly found that a monoester or diester of a hydroxy dicarboxylic acid can be advantageously used as an enhancer and more specifically as a skin permeation enhancer in a transdermal delivery system or a transdermal therapeutic system for increasing the *in vivo* flux of estrogens and more specifically of ethinyl estradiol from such transdermal delivery system or a transdermal therapeutic system to a subject such as a human being to which such transdermal delivery system or a transdermal therapeutic system has been applied. An *in vivo* flux for ethinyl estradiol of about 5 to 45 µg/24 hours, preferably of about 10 to 35 µg/24 hours more preferably of about 20 µg /24 h can thus be reached for a transdermal patch comprising a backing layer and a matrix layer. In an embodiment such transdermal delivery system and transdermal therapeutic system, respectively, typically has a substance weight of about 75 g/m² and contains a pharrmaceutically effective amount of ethinyl estradiol, wherein the amount of ethinyl estradiol is about 0.75 mg ethinyl estradiol or less, preferably about 0.65 mg ethinyl estradiol or less and more preferably about 0.60 mg ethinyl estradiol or less, whereby the surface area of the transdermal delivery system is about 20 cm². In an embodiment thereof, the flux of ethinyl estradiol is about 20 µg/24 hours. These figures are clearly indicative that the transdermal delivery system and the transdermal therapeutic system, respectively, according to the present invention are clearly superior to the ones of the prior art.

Because of such an increased *in vivo* flux the transdermal delivery system or a transdermal therapeutic system containing such enhancer shows a number of advantages.

Among others, the transdermal delivery system containing an enhancer according to the present invention may contain less of the pharmaceutically active agent such as a hormone and ethinyl estradiol in particular, or a lower content thereof, compared to a transdermal delivery system containing either no such enhancer or an enhancer different from the one of the present invention.

From such a compared to the prior art reduced content of the pharmaceutically active agent an improved stability of the transdermal delivery system arises as, at least for most pharmaceutically active agents including ethinyl estradiol, stability and content and concentration, respectively, are correlated in an inverse manner. At least part of the observed increase in stability at reduced concentrations arises from the absence of or reduction in crystallization of the pharmaceutically active agent which would otherwise reduce the *in vivo* flux. This means that the lower the content of the pharmaceutically active agent is, the more stable is the pharmaceutically active agent. This also means that the content of any stabilizer, if required, can also be reduced which is both making the manufacture of the transdermal delivery system easier and exposing the subject to which the transdermal delivery system is applied, to less chemicals.

The transdermal delivery system containing an enhancer according to the present invention showing an increased *in vivo* flux of the pharmaceutically active agent will also allow an easier disposal thereof as after the use of the transdermal delivery system the remaining amount and concentration, respectively, of the pharmaceutically active agent in the transdermal delivery system will be reduced. Apart from the aspect of easier disposal, also the ecological advantage which arises from the reduced content of the pharmaceutically active agent in the transdermal delivery system has to be acknowledged which applies equally to the aspect of a reduced content of stabilizer, if any, in the transdermal delivery system according to the present invention.

A still further advantage of the transdermal delivery system according to the present invention containing a compared to the prior art reduced content or amount of a pharmaceutically active agent and ethinyl estradiol in particular and providing an increased *in vivo* flux of the pharmaceutically active agent, is that such reduced content of the pharmaceutically active agent goes along with an increased stability of the pharmaceutically active agent which in turn provides for an even and constant flux of the pharmaceutically active agent. Such even and constant flux of the pharmaceutically active agent, however, is clearly advantageous and desirable from a therapeutic point of view with regard to the systemic levels of the pharmaceutically active agent and their generation in the subject to which the transdermal delivery system is administered.

It is to be acknowledged that the considerations are set forth herein in relation to transdermal delivery systems, but they equally apply to transdermal therapeutic systems, if not explicitly indicated to the contrary.

The enhancer according to the present invention can, in an embodiment of both the first and the second aspect of the invention which is an embodiment of each and any embodiment of the first and the second aspect of the present invention be a single species of compound. In an alternative embodiment which is an embodiment of both the first and the second aspect of the invention and which is an embodiment of each and any embodiment of the first and the second aspect of the present invention, the enhancer according to the present invention can be a mixture or composition of a plurality of chemical compounds or chemical species provided that the specific enhancer of the present invention is still contained. In a still further embodiment which is an embodiment of both the first and the second aspect of the invention and which is an embodiment of each and any embodiment of the first and the second aspect of the present invention, the enhancer according to the present invention comprises a plurality of chemical compounds or species provided that all of said compounds and species, respectively, share the feature that they are a monoester or a diester of a single or several hydroxy dicarboxylic acids as further defined herein. In the latter embodiment, a subgroup of enhancers are those where the different chemical compounds or species differ with regard to the alcohol moiety but have the same hydroxyl dicarboxylic acid backbone or moiety.

It will be acknowledged by a person skilled in the art that in case the enhancer according to the present invention is a single chemical compound or a mixture of chemical compounds which all share the backbone of a single or various hydroxy dicarboxylic acids, preferably a single hydroxy carboxylic acid, will be easier to manufacture compared to the transdermal delivery systems of the prior art comprising as an enhancer a mixture of different chemical compounds. Also from an analytical point of view, the use of a single chemical compound or a group of chemical compounds which have the same hydroxy dicarboxylic acid backbone or moiety is preferred. Furthermore, just like in the case of a reduced amount of a stabilizer or the omission of a stabilizer in the transdermal delivery system according to the present invention, these embodiments expose the subject to which the transdermal delivery system is applied to a reduced number of compounds which, among others, reduces the risk of allergic reactions in a subject to which the transdermal delivery system is applied.

One of the preferred compounds which can be used as an or the enhancers according to the present invention is COSMACOL^{®} EMI. COSMACOL ^{®} EMI is a di-ester obtained by combining malic acid and Isalchem 123, the latter being a mixture of several monobranched primary fatty alcohol isomers having hydrocarbon chain length with 12 and 13 carbon atoms. Its molecular structure is as follows: 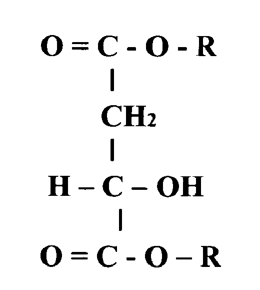 with R = C₁₂H₂₅ - C₁₃H₂₇,whereby the ratio between C_{12H25} and C₁₃H₂₇ is1:1.

The CAS registry no. of COSMACOL^{®} EMI is 149144-85-4, its INCI name is di-C12-C13 alkyl malate.

The physical properties of COSMACOL^{®} EMI are known to a person skilled in the art.

In a preferred embodiment of the present invention an enhancer is a skin permeation enhancer. A skin permeation enhancer is generally understood as an agent which assists and/or promotes the penetration and/or permeation of a further chemical compound such as a pharmaceutically active agent into the skin of a subject such as a mammal and more specifically of man, particularly if the enhancer and the further chemical compound are applied at the same time or concurrently. In connection therewith, preferably, penetration means the entering of said further chemical compound and more preferably of the pharmaceutically active agent, into the skin, and, preferably, permeation means the passage of the further chemical compound and preferable of the pharmaceutically active agent, through the skin whereby, as a final step, resorption of the further chemical compound may occur (see also Müller RH and Hildebrand GE, "Pharmazeutische Technologie: Moderne Arzneiformen", 2nd edition, Wissenschaftliche Verlagsgesellschaft mbH Stuttgart 1998, pages 141 - 142). Enhancers are, e.g., described in Walker RB and Smith EW, Advanced Drug Delivery Reviews 18 (1996) 295 - 301. Methods for the determination of whether or not a chemical compound or a mixture of chemical compounds is active as an enhancer are known in the art.

In a preferred embodiment of the transdermal delivery system according to the present invention which is also an embodiment of each and any embodiment of the third aspect of the present invention, the transdermal delivery system is a transdermal patch.

The basic design of transdermal delivery systems such as transdermal patches are known to the persons skilled in the art. Typically such transdermal delivery system comprises a backing layer, a matrix layer and a release liner. Preferably, the matrix layer is, on one side, affixed to the backing layer, and, on another side, to the release liner thus providing for a multi-layered design.

The backing layer and methods for producing it are known to the persons skilled in the art. The backing layer is substantially impermeable to skin permeation enhancing agents such as the enhancer according to the present invention. The backing layer can be made of any suitable material that is impermeable to the pharmaceutically active agents such as hormones and other excipients of the matrix layer. The backing layer serves as a protective cover for the matrix layer and provides a support function. The backing layer can be formed so that it is essentially the same size as the matrix layer containing the pharmaceutically active agent. The backing layer can be of any appropriate thickness that will provide the desired protective and support functions. A suitable thickness is from about 10 µm to about 300 µm. More specifically, the thickness is less than about 150 µm, yet more specifically, it is less than about 100 µm, and most specifically, the thickness is less than about 50 µm.

Examples of materials suitable for making the backing layer are films of acrylate, acrylonitrile-butadiene-styrene, acrylonitrile (methyl methacrylate) copolymer, acrylonitrile copolymer, ethylene ethyl acrylate, ethylene methyl acrylate, ethylene vinyl acetate, ethylene vinyl acetate copolymer, ethylene vinyl alcohol polymer, ionomers, nylon (polyamide), nylon (polyamide) copolymer, polybutylene, polycarbonate, polyester, polyethylene terephthalate, thermoplastic polyester,copolymer, polyethylene copolymer (high density), polyethylene (high-molecular-weight, high density), polyethylene (intermediate-molecular weight, high density), polyethylene (linear, low density), polyethylene (low density), polyethylene (medium density), polyethylene oxide, polyimide, polypropylene, polypropylene (coated), polypropylene (oriented), polystyrene, polyurethane, polyvinyl acetate, polyvinyl chloride, polyvinylidene chloride and/or styrene-acrylonitrile. It is within the present invention that such films may be metallised or pigmented. Preferred materials for the manufacture of the backing layer are polyurethane, ethylene vinyl alcohol polymer and polyester.

The release liner is to protect the adhesive which mediates the attachment of the transdermal delivery system to the subject to which the transdermal delivery system is applied whereby the adhesive may form a separate layer or be part of the matrix layer. The release liner is thus to be removed from the transdermal delivery system prior to the application to the subject.

Release liners and methods for producing it are known to the persons skilled in the art. Release liners can be formed of polyester, polyethylene, polypropylene, polysiloxane, e.g. with a fluorosiliconized coating, polyacrylate, ethylene vinyl acetate, polyurethane, polyisobutene or paper. Preferably the paper is coated with silicone and/or polyethylene. In an embodiment, a foil consisting of polyethylene terephthalate is used, whereby, preferably, one side of such foil is siliconized. Preferably, the thickness of such release line is about 75 µm. Also a combination of any of the above materials may be used in the preparation of the release liner. The release liner preferably also comprises an adhesive which may be one as defined herein.

The matrix layer and methods for producing it are known to the persons skilled in the art. The matrix layer actually hosts the pharmaceutically active agent such as a hormone, preferably in an effective amount. Preferably, the pharmaceutically active agent is dispersed or dissolved in said matrix layer, preferably evenly dispersed or dissolved therein. It is evident that the matrix layer must allow the transfer of the pharmaceutically active agent from the matrix layer into the skin to which the transdermal delivery system is applied. The matrix layer may by formed by polyacrylate, polyisobutylene, silicones, styrene block copolymers such as styrene isoprene block copolymer (SIS), or a mixture thereof.

The matrix layer may comprise further constituents such as solubilizers. Such solubilizers increase the solubility of the pharmaceutically active agent. Solubilizers for use in transdermal delivery systems are known to the skilled person of the art. Solubilizers which may be used in the transdermal delivery agent according to the present invention include but are not limited to soluble polyvinyl pyrrolidones suchas Kollidon^{®}.

A preferred embodiment of Kollidon^{®} are the different Kollidon^{®} CL grades which can best be distinguished by their different particle sizes. More specifically, the average particle size range [µm] is as follows: Kollidon^{®} CL: 110-130; Kollidon^{®} CL-F: 20-40; Kollidon^{®} CL-SF: 10-30; Kollidon^{®} CL-M: 3-10. All CL-grades are crosslinked, water-insoluble polivinyl pyrrolidones. There are chemical but mainly physical crosslinks. Chemical names for Kollidon which are used in an interchangeable manner, are as follows: Crospovidone; Crospovidonum; insoluble polyvinylpyrrolidone; and crosslinked PVP. In contrast to many other disintegrants, the Kollidon^{®} CL grades are non-water-soluble.

The matrix layer may also further comprise fillers. Fillers for use in transdermal delivery systems are known to the skilled person of the art. Fillers which may be used in the transdermal delivery agent according to the present invention include but are not limited to silicon dioxide, polyvinyl pyrrolidone, cross-linked polyvinyl pyrrolidone (crospovidone), Kollidon^{®} CL-M, metal oxides such as titanium dioxide or zinc dioxide, talc, silicates such as magnesium silicate or aluminium silicate, stearates such as zinc stearate, polyethylene, polystyrene, and mixtures thereof. A preferred filler is crospovidone. Typically, the function of the filler is such that it acts as a crystallization inhibitor and/or that it increases the mechanical stability of the transdermal therapeutic system.

The transdermal delivery system according to the present invention may also comprise an adhesive, whereby such adhesive may either form a further layer which is interposed between the matrix layer containing the pharmaceutically active agent and the enhancer and the release liner, or it may be contained in the matrix layer thus providing for an adhesive matrix layer. In a preferred embodiment the adhesive is a pressure-sensitive adhesive.

In a preferred embodiment, the transdermal delivery system of the invention contains only one adhesive.

Adhesives and more specifically pressure-sensitive adhesives for use in transdermal delivery systems are known to the skilled person of the art and, e.g., described in the Handbook of Pressure Sensitive Adhesive Technology, 2nd Edition (1989) Van Nostrand, Reinhold. Adhesives and more specifically pressure-sensitive adhesives which may be used in the transdermal delivery system according to the present invention include but are not limited to polyacrylate, silicones and polyisobutylene (PIB). A preferred adhesive is polyisobutylene.

Polyisobutylene adhesives are preferably mixtures of high molecular weight (HMW) PIB and low molecular weight (LMW) PIB. Such mixtures are described in the art, e.g., PCT/US91/02516. The molecular weight of the HMW PIB will usually be in the range of about 700,000 to 2,000,000 Da, whereas that of the LMW PIB will typically range between 35,000 to 60,000. The molecular weights referred to herein are weight average molecular weight. The weight ratio of HMW PIB to LMW PIB in the adhesive will normally range between 1: 1 to 1:10. The PIB adhesive may also include a tackifier such as polybutene oil and high Tg, low molecular weight aliphatic resins such as the ESCOREZ^{™} resins available from Exxon Chemical. Polyisobutylene polymers are commercially.

Examples of polyisobutylene useful for such purpose are the so-called DUROTAK^{®} s.

One embodiment of the DUROTAK^{®}s is DURO-TAK 87-608 A which is a solution in heptane of polyisobutylene rubber and polybutene rubber the total solid content of which is 38 to 40 % (w/w). The polyisobutylene rubber makes up 85 % of total solids, and polybutene rubber makes up 15 % of total solids. The viscosity is 2,000 - 10,000 cps (according to Brookfield, determined at 22.2°C, i.e. 72° Fahrenheit, at 20 rpm, using as spindle Spindle No. 4).

Another embodiment of the DUROTAK^{®}s is DURO-TAK 87-6430 which has a viscosity of about 6,000 to 14,000 cps (according to Brookfield, determines at 22.2°C, i.e. 72 ° Fahrenheit, at 20 rpm, using as spindle Spindle No. 4) and which is a solution in heptane of polyisobutylene rubber and polybutene rubber the total solid content of which is 27 to 33% (w/w). The polyisobutylene rubber makes up 90 % of total solids, and polybutene rubber which makes up 10 % of total solids.

In a still further embodiment, a pressure-sensitive adhesive may be used such as the so-called oppanoles which are either used as a single compound or as a mixture of several of such compounds, and also belong to the group of polyisobutylenes. Some of such oppanoles and their characteristics are summarized in the following tables 3 and 4.2:

| Table 3. Commercial grades of polyisobutylene commonly used in pressure-sensitive adhesive^{15,16} | | | |
|---|---|---|---|
| Supplier | Product name | Grade | Viscosity average molecular weight |
| | | | |
| Exxon | Vistanex^{®} | LM-MS | 43,000-46,000 |
| | | LM-MH | 51,000-56,000 |
| | | LM-H | 58,000-68,000 |
| | | MM L-80 | 750,000-1,050,000 |
| | | MM L-100 | 1,060,000-1,440,000 |
| | | MM L-120 | 1,450,000-1,870,000 |
| BASF | Oppanol^{®} | B10 | 40,000 |
| | | B12 | 55,000 |
| | | B15 | 85,000 |
| | | B30 | 200,000 |
| | | B50 | 400,000 |
| | | B100 | 1,110,000 |
| | | B150 | 2,600,000 |

**TABLE 4.2 Commercial Grades of PIBs**

| Name | Grade | Viscosity Average Molecular Weight (*M*,/g/mol) | Comments |
|---|---|---|---|
| BASF Oppanol B | 10 SNF | 40,000 | Soft, resin-like polymers, used in the |
| | 11 SNF | 49,000 | production of adhesives, sealants, |
| | 12 SNF | 55,000 | lubricants, coatings, and chewing gum |
| | 13 SNF | 65,000 | |
| | 15 SNF | 85,000 | |
| | 30 SF | 200,000 | Soft, resin-like polymers, used for |
| | 50/50 SF | 400,000 | producing adhesives, sealants, lubricating |
| | 80 | 800,000 | oils, coating compounds, and chewing |
| | | | gum; also recommended for modifying |
| | | | bitumen |
| | 100 | 1,100,000 | Rubbery polymers, used for the |
| | 150 | 2,600,000 | production of adhesives, sealants, |
| | 200 | 4,000,000 | lubricants, and coating compounds |

In another embodiment, pressure sensitive solution polyacrylate adhesives are made by copolymerizing one or more acrylate monomers ("acrylate" is intended to include both acrylates and methacrylates), one or more modifying monomers, and one or more functional group-containing monomers in an organic solvent. The acrylate monomers used to make these polymers are normally alkyl acrylates of 4-17 carbon atoms, with 2-ethylhexyl acrylate, butyl acrylate, and isooctyl acrylate being preferred. Modifying monomers are typically included to alter the Tg of the polymer. Such monomers as vinyl acetate, ethyl acrylate and methacrylate, and methyl methacrylate are useful for this purpose. The functional group-containing monomer provides sites for crosslinking. The functional groups of these monomers are preferably carboxyl, hydroxy or combinations thereof. Examples of monomers that provide such groups are acrylic acid, methacrylic acid and hydroxy-containing monomers such as hydroxyethyl acrylate. The polyacrylate adhesives are preferably crosslinked using a crosslinking agent to improve their physical properties, (e.g., creep and shear resistance). The crosslinking density should be low since high degrees of crosslinking may affect the adhesive properties of the copolymer adversely. Examples of crosslinking agents are disclosed in US Pat. No. 5,393,529. Solution polyacrylate pressure sensitive adhesives are commercially available under tradenames such as GELVA ^{™} and DURO-TAK^{™}.

The silicone adhesives that may be used in forming the matrix are typically high molecular weight polydimethyl siloxanes or polydimethyl diphenyl siloxanes. Formulations of silicone adhesives that are useful in transdermal patches are described in U.S. Patent Nos. 5,232,702, 4,906,169 and 4,951,622.

The matrix layer may further contain one or several stabilizers. Such stabilizers increase the stability of the pharmaceutically active agent which is preferably contained in the matrix layer. More specifically, and preferably the function of such stabilizers is to inhibit the cold-flow of an adhesive. Such stabilizers for use in transdermal delivery systems are known to the skilled person of the art and include, but are not limited to silicon dioxide, polyvinyl pyrrolidone, cross-linked polyvinyl pyrrolidone (crospovidone), Kollidon^{®} CL-M.

Finally the matrix layer may comprise as a further constituent one or more tackifiers. Such tackifiers increase the adhesiveness of the matrix layer, preferably of the adhesive matrix layer. Tackifiers for use in transdermal delivery systems are known to the skilled person of the art. Tackifiers which may be used in the transdermal delivery agent according to the present invention include but are not limited to resins such as colophony resin, phenol resins, alkylphenol resins, petroleum resins and xylene resins, or mixtures thereof.

It is to be understood that any percentage indicated herein is wt%. If a weight percentage is said to be a wt% of, e.g., the matrix layer, then said wt% is a wt% of the weight of the matrix layer.

Also, it is to be understood, that as used herein a wording defining the limits of a range of length such as e. g. "from 1 to 5" or "1- 5" means any integer from 1 to 5, i. e. 1, 2, 3, 4 and 5. In other words, any range defined by two integers explicitly mentioned is meant to comprise and indicate any integer defining said limits and any integer comprised in said range.

The transdermal delivery agent according to the present invention may, in its various embodiments, be used for preventing ovulation in a woman, i.e. a female human being. It will be acknowledged by a person skilled in the art that basically a variety of hormones or combination of hormones may be administered to a woman for such purpose, including administration to such woman by a transdermal delivery system such as a transdermal patch also according to the present invention. A particular preferred combination of hormones for such purpose is the combination of 17-deacetyl norgestimate and ethinyl estradiol. Such combination is advantageous because of its positive effect on the female metabolism. Among others, the level of the high-density lipoprotein is increased and the ratio in serum of low-density lipoprotein to high-density lipoprotein is increased.

For the purpose of preventing ovulation in a woman, the amount of 17-deacetyl norgestimate administered through the transdermal delivery system according to the present invention is about 100 - 300 µg/day, preferable about 150 µg/day. The amount of ethinyl estradiol administered through the transdermal delivery system according to the present invention is about 10 - 35 µg/day, preferable about 20 µg/day. The preferred combination of ethinyl estradiol and 17-deacetyl norgestimate which is administered to a woman for the purpose of preventing ovulation is thus 20 µg/day of ethinyl estradiol and 150 µg/day of 17-deacetyl norgestimate by means of the transdermal delivery system according to the present invention. Preferably the transdermal delivery system according to the present invention is a patch, whereby the patch is intended to be worn for about 1 to 7 days, preferably 7 days.

The transdermal system according to the present invention, also when used for preventing ovulation in a woman, is preferably a transdermal patch of about 10 to 50 cm². So as to make sure that the anti-ovulation effect is taking place the transdermal delivery system is applied to the woman starting from day 1 of the menstruation cycle to day 21 of the menstruation cycle. If the individual transdermal delivery system and more specifically the transdermal patch is worn for 7 days, the first patch has to be replaced by a second one, and possibly by one or more further transdermal patches so as to ensure an ongoing delivery of the hormone(s) over the period of time requires so as to prevent the ovulation in the woman.

The transdermal delivery agent according to the present invention may, in its various embodiments, also be used for providing hormone replacement therapy in a woman. It will be acknowledged by a person skilled in the art that basically a variety of hormones or combination of hormones may be administered to a woman for such purpose, including administration to such woman by a transdermal delivery system such as a transdermal patch, also according to the present invention. One hormone which is preferably administered for such purpose is 17-deacetyl norgestimate which is more preferably administered together with an effective amount of an estrogen. Such estrogen is preferably either ethinyl estradiol or 17-ß-estrogen.

For the purpose of providing hormone replacement therapy to a women, the amount of 17-deacetyl norgestimate administered through the transdermal derlivery system and more specifically the transdermal patch according to the present invention to the woman is about 150 - 350 µg/day, preferable about 175 - 300 µg/day. The amount of ethinyl estradiol administered through the transdermal derlivery system and more specifically the transdermal patch according to the present invention to the woman is about 5 - 45 µg/day, preferable about 10 - 35 µg/day.

A preferred administration of hormones by means of the transdermal delivery system and more specifically the transdermal patch according to the present invention is a combination of 17-deacetyl norgestimate of which about 175 - 300 µg/day are administered to the woman, and ethinyl estradiol of which about 10 to 35 µg/day are administered to the woman by means of said transdermal patch.

An alternative preferred administration of hormones by means of the transdermal delivery system and more specifically the transdermal patch according to the present invention is a combination of 17-deacetyl norgestimate of which about 175 - 300 µg/day are administered to the subject, and 17-ß-estradiol of which about 30 to 150 µg/day are administered to the subject by means of said transdermal patch.

The transdermal system according to the present invention, also when used for providing hormone replacement therapy in a woman, is preferably a transdermal patch of about 10 to 50 cm². So as to make sure that the hormone replacement effect is taking place the transdermal delivery system is applied to the woman for the duration of the hormone replacement therapy. If the individual transdermal delivery system and more specifically the transdermal patch has been worn for a period of typically 1 to 7 days, the patch preferably has to be replaced by one or more further transdermal patches so as to ensure an ongoing delivery of the hormone(s) over the period of time required for the hormone replacement therapy.

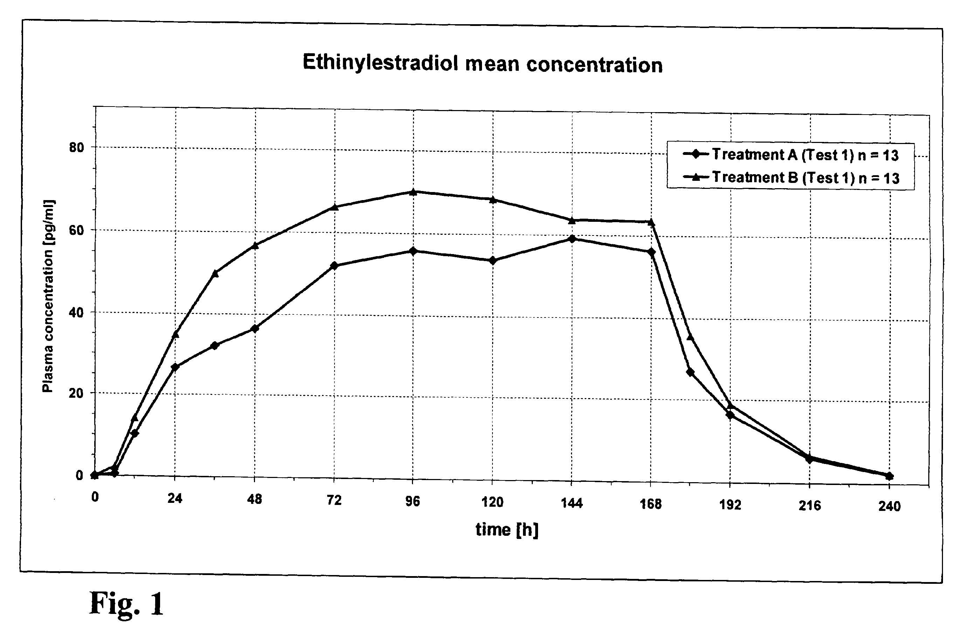

The present invention is now further illustrated by the following figures and examples from which further advantages, features and embodiment may be taken, whereby
- Fig. 1: is a diagram indicating plasma concentration of ethinyl estradiol (pg/ml) over time (h) upon application to healthy female adults of a transdermal patch according to the present invention and a transdermal patch according to the prior art.

### Example 1: Preparation of transdermal patches containing 17-deacetyl norgestimate

The various transdermal patches were prepared as follows.

17-deacetyl norgestimate and ethinyl estradiol were dissolved in a mixture of 2-propanol and ethylacetate (1:1) providing the drug containing solution. COSMACOL® EMI and Kollidon^{®} CL-M were added to the drug containing solution and the thus obtained suspension was homogenized, typically at a temperature between 20 °C and 25 °C using a magnetic stirrer. Polyisobutylene dissolved in heptane (Durotak 87-608A) acting as an adhesive was added and the thus obtained mixture was further homogenized.

The thus obtained suspension was applied to the siliconized side of a film which acted as the release liner. Such release liner consisted in the instant cases of a transparent PET-foil having a thickness of about 75 µm. The thus one-side coated release liner was dried in a drying channel. Upon drying, the matrix layer was formed. A PET film (Hostaphan RN 19) was placed on the matrix layer as the drug impermeable backing layer. The transdermal patches were subsequently punched from the thus obtained layered product.

The following transdermal patches ("TTS") were produced in accordance with the above.

### Patch A:

| Compound | Amount [mg]/20 cm² TTS |
|---|---|
| Ethinyl estradiol | 0.75 |
| 17-deacetyl norgestimate | 6 |
| Isopropyl palmitate | 11.25 |
| Kollidon CLM | 30 |
| Polyisobutylene (Durotak 87-608A) | 102* |

| | |
|---|---|
| *expressed as solid matter content of the solution; solvents are removed during manufacturing Substance weight: 75 g/m² | |

### Patch B:

| Compound | Amount [mg]/20 cm² TTS |
|---|---|
| Ethinyl estradiol | 0.75 |
| 17-deacetyl norgestimate | 6 |
| COSMACOL^{®} EMI | 11.25 |
| Kollidon CLM | 30 |
| Polyisobutylene (Durotak 87-608A) | 102* |

| | |
|---|---|
| *expressed as solid matter content of the solution; solvents are removed during manufacturing Substance weight: 75 g/m² | |

As is evident from the above compositions of patches A and B contain the same absolute content of ethinyl estradiol per surface area, however exhibit different enhancers with patch A containing the enhancer isopropyl palmitate as known, e.g., from WO 96/40355 which is an ester of a monocarboxylic acid. Patch B contains as the enhancer COSMACOL ^{®} EMI.

Accordingly, patch A is a patch of the prior art and thus acts as a comparative patch not falling within the scope of the claims.

### Example 2: In vivo studies on plasma levels of ethinyl estradiol upon application of patches A and B

In order to determine the transdermal delivery characteristics of transdermal patches A and B as prepared according to Example 1, the following study was performed.

### Study design:

The study was a single dose, 2-way, crossover, randomized study on n = 16 healthy females. Application period was 7 days. Between the various periods of administration of one of patches A and B, a three week wash-out period was maintained.

Treatment A used patch A and treatment B used patch B.

Samples from the individuals participating in the study were taken in accordance with standard procedures known to the ones skilled in the art.

Analysis of ethinyl estradiol was performed in accordance with standard procedures known to the ones skilled in the art .

The results of study are shown in Fig. 1.

As may be taken from Fig. 1 treatment B which uses a transdermal patch according to the present invention clearly provides an increased plasma concentration of ethinyl estradiol than treatment A using patch A which is a transdermal patch of the prior art. Apart from an increase in plasma level, such plasma level is reached faster and the level shows a more pronounced plateau phase which is clearly advantageous. Both patch A and B contain the same amounts for the various ingredient and different only in the enhancer.

The features of the present invention disclosed in the specification, the claims and/or the drawing may both separately and in any combination thereof be material for realizing the invention in various forms thereof.

## Claims

1. An enhancer consisting of a monoester or diester of a hydroxy dicarboxylic acid, for use in the transdermal administration of a pharmaceutically active agent to a subject.

2. The enhancer according to claim 1, wherein the hydroxy dicarboxylic acid is of formula (I)
R₁OOC-(CHOH)ₓ-(CH₂)_{y}-(CH₂OH)₂-COOR₂ (I)
wherein x and z are each and independently any integer from 0 to 5, whereby if x = z, x and z are ≠ 0 ,
preferably x is any integer from 1-5 ,
more preferably x is 1 or 2, and z is 0 ;
y is any integer form 0 to 10, preferably 1-5 , most preferably 1; and
R1 and R2 are each and independently selected from the group consisting of H, C₁-C₈, C₈-C₁₈, wherein at least one of R₁ and R₂ has to be different from H.

3. The enhancer according to claim 2, wherein R₁ and R₂ are each and independently a mixture of a branched C₁₂ and C₁₃ alcohol of formula (II) 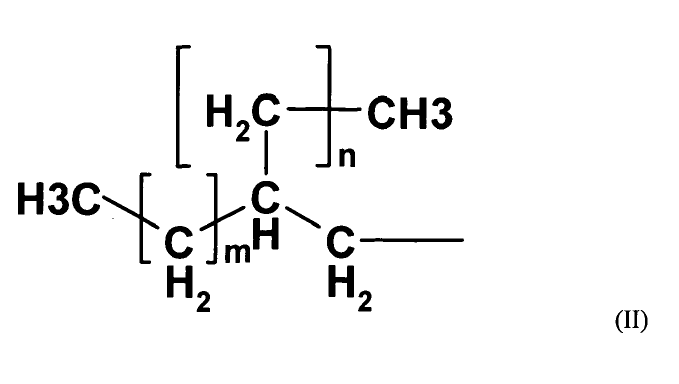 wherein m + n is either 8 or 9.

4. The enhancer according to any one of claims 1 to 3, preferably claim 3, wherein the hydroxy dicarboxylic acid is selected from the group comprising 2-hydroxybutanedioic acid and 2,3-dihydroxybutanedioic acid.

5. The enhancer according to any one of claims 1 to 4, wherein the enhancer is COSMACOL^{®} EMI.

6. The enhancer according to any one of claims 1 to 5, wherein the pharmaceutically active agent comprises at least one hormone.

7. The enhancer according to claim 6, wherein the at least one hormone is one selected from the group consisting of 17-deacetyl norgestimate, ethinyl estradiol and 17-ß-estradiol, preferably the pharmaceutically active agent comprises or consists of a combination of 17-deacetyl norgestimate and ethinyl estradiol.

8. A transdermal delivery system comprising
a) a backing layer,
b) a matrix layer underlying the backing layer, the matrix layer comprising a pharmaceutically active agent, and a skin permeation enhancer, and
c) optionally a release liner, and
d) further optionally at least one adhesive layer,
wherein the skin permeation enhancer is an enhancer as defined in any one of claims 1 to 5.

9. The transdermal delivery system according to claim 8, wherein the transdermal delivery system comprises at least one adhesive in the matrix layer.

10. The transdermal delivery system according to any one of claims 8 to 9, wherein the pharmaceutically active agent comprises at least one hormone, preferably an estrogen and more preferably ethinyl estradiol or 17-ß-estradiol.

11. The transdermal delivery system according to any one of claims 8 to 10, wherein the transdermal delivery system comprises:
a) a backing layer,
b) a matrix layer underlying the backing layer; and
c) a release liner,
wherein the matrix layer contains ethinyl estradiol, 17-deacetyl norgestimate, Cosmacol EMI, an adhesive, and optionally cross-linked polyvinylpyrrolidon, wherein preferably the adhesive is polyisobutylene.

12. The transdermal delivery system according to any one of claims 8 to 11, wherein the transdermal delivery system comprises:
a) a backing layer,
b) a matrix layer underlying the backing layer; and
c) a release liner,
wherein the matrix layer contains ethinyl estradiol, 17-deacetyl norgestimate, Cosmacol EMI, Kollidon CLM and Durotak 87-608A, wherein the amount of ethinyl estradial contained in the matrix layer is a pharmaceutically active amount of ethinyl estradiol of about 0.75 mg/20 cm² or less, preferably of about 0.65 mg/20 cm² and more preferably of about 0.6 mg/20 cm², and wherein the surface weight of the transdermal delivery system is 75 g/m².

13. The transdermal delivery system according to any one of claims 1 to 12, wherein the matrix layer contains
| Compound | Amount [mg/20 cm² of the transdermal delivery system] |
|---|---|
| Ethinyl estradiol | 0.75 |
| 17-deacetyl norgestimate | 6 |
| COSMACOL^{®} EMI | 11.25 |
| Kollidon CLM | 30 |
| Polyisobutylene (solid matter), preferably Durotak 87-608A | 102 |
and wherein the surface weight of the transdermal delivery system is 75 g/m².

14. The transdermal system according to any one of claims 8 to 13 for use in inhibiting ovulation in a woman or for use in providing hormone replacement therapy to a woman.

15. Use of an enhancer according to any one of claims 1 to 7, in the manufacture of a medicament or a pharmaceutical formulation, whereby, preferably, the medicament or pharmaceutical formulation is a transdermal delivery patch comprising at least one pharmaceutically active agent.
